# EUROPÄISCHE PATENTANMELDUNG

(11) **EP 0 779 073 A1**
(43) Veröffentlichungstag der Anmeldung: **18.06.1997**
(21) Anmeldenummer: 96119816.5
(22) Anmeldetag: 10.12.1996
(51) Int. Cl.: A61K 9/70, A61K 31/165, A61K 31/245

(54) **Methode und Zusammensetzung einer topisch wirksamen Therapie postoperativer und posttraumatischer Wundschmerzen**

(30) Priorität: 11.12.1995 DE 19546159
(71) Anmelder: Liedtke, Rainer K., Dr., D-82027 Grünwald (DE)
(72) Erfinder: Liedtke, Rainer K., Dr., D-82027 Grünwald (DE)
(74) Vertreter: Bühling, Gerhard, Dipl.-Chem.

(57) **Zusammenfassung**

Als Therapie postoperativer oder posttraumatischer Wundschmerzen werden geeignete topisch wirksame Trägersysteme eingesetzt, die mit therapeutisch geeigneten Dosen von Lokalanästhetika beladen sind und die enthaltenen Lokalanästhetika topisch gezielt in, auf oder an das Wundgebiet bringen, aus biologischen oder künstlichen Materialien oder einer Kombination von beidem bestehen sowie über zusätzliche Mechanismen zur Kontrolle der Freisetzung der enthaltenen Lokalanästhetika verfügen können und wobei sich das zu therapierende Wundgebiet entweder im Inneren des Körpers oder an der Körperoberfläche befindet.

Die Therapie mit geeigneten topisch wirksamen Trägersystemen mit Lokalanästhetika stellt eine neue nichtinasive, nebenwirkungsarme und effektive Behandlungsmöglichkeit postoperativer oder posttraumatischer Wundschmerzen dar, die neben den medizinischen Vorteilen auch kostensparende Therapievorteile ermöglicht.

## Beschreibung

Die Erfindung betrifft die Methode und Zusammensetzung einer topisch wirksamen Therapie postoperativer und posttraumatischer Wundschmerzen.

Es ist bekannt, daß nach physikalischer Verletzung, z.B. mechanischer oder thermischer Art, oder als Folge der Intervention einer chirurgischen Operation Gewebstraumata entstehen und hierbei Schmerzen, häufig auch Entzündungen, als übliche biologische Reaktionen auftreten.

Bei chirurgischen Operationen ist seitens der Patienten die subjektiv am meisten gefürchtete Folge der postoperative Schmerz. Dies gilt sowohl für den stationären wie auch den ambulanten Bereich. Gleiches gilt auch für das Auftreten von Schmerzen infolge Unfall-Traumata, die insbesondere bei geschlossenen Verletzungen u.a. auch Alltags- oder Sport-Verletzungen wie z.B. Verstauchungen, Überdehnungen, Prellungen, Kontusionen, Luxationen, Subluxationen, sehr lange anhalten.

Der Schmerz wird im wesentlichen durch mechanische und konsekutiv Irritationen der sensorischen Innervation ausgelöst und kann z.B. u.a. durch Phasen reaktiver Ödembildungen oder auch Gewebshormone verstärkt werden. Diese Vorgänge können auf neuronalem Wege, durch interne neuronale Verschaltungen, noch zu einer weiteren schmerzsteigernden Circulus Vitiosus Charakteristik führen und sich hierüber, vom Ursprungstrauma weitgehend unabhängig, selbst noch weiter aufrechterhalten und damit chronifizieren.

Unabhängig von der Art der mechanischen Intervention, sei es durch operativen Eingriff oder anderweitiges Trauma, ist der regeltechnische Verlauf einer Schmerzsymptomatik daher überwiegend gleich. Eine Gemeinsankeit besteht auch insoweit, daß stets auch verschiedene sekundär neurologische, muskuläre, fasculäre Symptomatiken einbezogen sind und in die traumatischen Schmerzvorgänge u.a. auch Infektionen, immunologische Prozesse, physikalische Umweltfaktoren wie auch psychosomatische Stressfaktoren hineinwirken können.

Postoperative oder postraumatische Schmerz-Symptomatiken werden weit überwiegend, mit systemisch wirksamen, nichtopioiden, oralen oder injektablen, Analgetika oder nichsteroidalen Antiphlogistika behandelt, partiell in Kombination mit psyschosomatischen oder physikalischen Behandlungen. In schweren Fällen werden zusätzlich auch Opioide oder Opiate eingesetzt. Als pharmakologische Prinzipien handelt es sich dabei insbesondere um Derivate der Salicysäure, vorzugsweise Stoffe wie Acetylsaliclsäure, sogenannte nichsteroidale Antiphlogistika, u.a. z.B. Ibuprofen, wie auch Anilin-Derivate, z.B. Paracetanol. Weiterhin erfolgt auch der Einsatz zentraler oder peripherer Muskelrelaxantien, gelegentlich der von Tranquillantien der Benzodiazepin-Gruppe wie auch der von Antidepressiva, in schweren Fällen der von Opioiden z.B. Pentacocin, Buprenorphin, Tramadal oder Opiaten (e.g. Brune K. Beck W. in: M. Zenz, I. Jura (Edits.) Lehrbuch der Schmerztherapie, WFG, Stuttgart 1993, S.121-135)

Systemische Pharmakotherapien stellen insgesamt noch keine ausreichend verträgliche und effektive Behandlungsform postoperativer oder postraumatischer Schmerzen dar, da deren Therapie auch eine für das Trauma zielgerechte Behandlungsmaßnahme erfordert. Eine systemische Verteilung der Wirkstoffe bezieht aber stets auch alle nichtbetroffenen Organe und Organsysteme des Körpers mit ein. Auf Grund der Akuität postoperativer und posttraumatischer Beschwerden sind zudem systemisch meist höhere Dosen erforderlich.

Systemisch analgetischen Therapien gemeinsam ist eine erhebliche Rate unerwünschter Wirkungen, insbesondere bei chronischer Anwendung. Bei nichtsteroidalen Antiphlogistika finden sich im Vordergrund erhebliche wie häufige Magenstörungen, u.a. Erosionen von Magen- und Darmschleimhaut oder Blutungen, als Folge ihres antiproliferativen Wirkungsmechanismus. Bei dem schwacher wirksamen Paracetamol, das keine antiphlogistische Wirkkomponente hat, findet sich demgegenüber eine metabolische Belastung der Leber- und Nieren-Funktionen, insbesondere bei dem erforderlich höher dosierten Gebrauch. Muskelrelaxantien beinhalten u.a. starke Sedierungen sowie zahlreiche andere zentrale Nebeneffekte, teils auch Ausbildung von Paresen. Opioide und Opiate beinhalten, neben unerwünschten Effekten auf u.a. die Respiration, ein hohes Abhängigkeitsrisiko.

Weiterhin sind bei all diesen Stoffen, bei systemischer Anwendung, auch noch zahlreiche pharmakologische Interaktionen mit anderen Wirkstoffen gegeben, was gerade in einer stationär oder ambulant postoperativen Phase von besonderer Bedeutung ist, da hier häufig multipel therapiert werden muss. Insbesondere zeigen viele dieser Wirkstoffe auch Einflüsse auf Blutungs- und Blutgerinnungs-Verhalten. Somit wird die Anwendung systemischer Therapien durch ihr jeweilige Spektrum spezifischer unerwünschter Wirkungen wie auch deren Interaktionen limitiert, da diese Anwendungen stets alle nichtbetroffenen Organe und Organsysteme des Körpers mit einbeziehen.

Ein Teil dieser Analgetika wird daher auch in Form topischer bzw. lokaler Applikationen, z.B. als Cremes, äußerlich versucht. Doch setzt deren Form des Wirkungsmechanismus prinzpiell auch eine geeignet hohe Penetration voraus, mit auch systemischen Wirkspiegeln, so daß in der Regel keine effektive Schmerztherapie erzielt wird.

Ein vom therapeutischen Ansatzpunkt weit vorteilhafteres pharmakologisches Prinzip wäre eine geeignete Form einer Anwendung niedrig dosierter Lokalalänästhetika, direkt im oder am Ort der Verletzung.

Amid- und Ester-Lokalanästhetika, z.B. Lidocain vom Amid-Typ, zeigen als pharmakologischen Wirkungsmechanismus vorrangig eine Hemmung des schnellen Natrium-Ionen Influx in Nervenfasern. Sie blockieren hierüber die Impulsleitung der Nervenbahn, was prinzipiell alle regionalen Nervenfasern betrifft. Die sensorischen, anatomisch dünneren, Fasern sind infolge ihrer Morphologie empfindlicher als motorische Fasern (Strichartz, G.R. (Ed.) Local Anesthetics, Handbook of Experimental Pharmacology, Vol. 81, Springer, Berlin/New York, 1987). Hieraus lassen sich auch Wirkeffekte auf sensorische, schmerzempfindliche Rezeptororgane differenzieren. Lokalanästhetika verfügen zudem über eine angemessen gute Gewebsverträglichkeit, haben in einigen Fällen, z.B. Lokalanästhtetika vom Amid-Typ, bakterizide Eigenschaften und wirken, infolge auch einer Beeinflussung regionaler Gefäßinnervation, auf Wundheilungs- und Entzündungsvorgänge eher positiv.

Eine systemische Anwendung von Lokalanästhetika wäre für diese Therapie nicht sinnvoll und entfällt schon weitgehend auf Grund der Gefahr systemischer Überdosierungen mit, u.a., ernsten kardialen Nebenwirkungen. Anwendungen von Lokalananästhetika mittels lokaler Injektionen sind technisch möglich und werden auch verschiedentlich ausgeführt. Doch sind lokale Injektionen mit Spritzen, als eine invasive Maßnahme, nicht nur schmerzhaft, sie können auch von den Patienten selbst nicht ausgeführt werden. Sie beinhalten zudem gleichfalls die Gefahr systemischer Intoxikation, und besitzen auch nur eine sehr begrenzte Wirkdauer. Eine äußerlich lokale Injektionstechnik beruht dabei u.a. auf der sogenannten Neuraltherapie mit Triggerpunkten und setzt eine erfahrene ärzliche Handhabung und Technik voraus (J.T. Travell, D.G. Simons, Myofascial Pain and Dysfunction, Vol I/II, Williams & Wilkins, Baltimore, 1983). Ein Einsatz konventioneller, topischer Formulierungen, z.B. von Cremes, erlaubt weder die für diese Wirkstoffe erforderliche exakte Dosierung noch eine kontinuierliche Wirkstoff-Penetration über längere Anwendungsdauer. Sie ermöglicht damit weder eine ausreichende Therapiesicherheit noch eine ausreichende oder kalkulierbare Wirkung.

Der Erfindung liegt die Aufgabe zugrunde die Therapie postoperativer und posttraumatischer Wundschmerzen zu verbessern.

Diese Aufgabe wird dadurch gelöst, daß als Therapie geeignete topisch wirksame Trägersysteme mit Lokalanästhetika eingesetzt werden, dadurch gekennzeichnet, daß diese mit therapeutisch geeigneten Dosen von Lokalanästhetika beladen sind, die die enthaltenen Lokalanästhetika topisch gezielt in, auf oder an das Wundgebiet bringen, diese aus biologischen oder künstlichen Materialien oder einer Kombination von beidem bestehen sowie über zusätzliche Mechanismen zur Kontrolle der Freisetzung der enthaltenen Lokalanästhetika verfügen können, und sich das zu therapierende Wundgebiet entweder im Inneren des Körpers oder an der Körperoberfläche befindet.

Um die Wirksamkeit und Verträglichkeit dieser topisch wirksamen Therapie zu verbessern sind, in einer weiteren Ausbildung der Erfindung Lokalanästhetika vom Amid- oder Ester-Typ enthalten, insbesondere Lidocain, Tetracain, Bupivacain, Prilocain, Mepivacain, Etidocain sowie Procain und Benzocain, wobei diese Stoffe in Konzentrationsbereichen von 0,5%-40% vorliegen.

Um die Wirksamkeit und Verträglichkeit dieser Therapie zu verbessern, werden, in einer weiteren Ausbildung der Erfindung, in dem eingesetzten topisch wirksamen Trägersytem 2 Lokalanästhetika mit unterschiedlicher Pharmakodynamik und Pharmakokinetik kombiniert, wobei diese Einzelstoffe in solchen Konzentrationen vorliegen, daß die Gesamkonzentration beider Wirkstoffe nicht mehr als 40% beträgt.

Um die Therapie insgesamt sicherer, anwendungsspezifischer und besser handhabbar zu machen, liegt, in einer weiteren Ausbildung der Erfindung, das topisch wirksame Trägersystem in solchen geometrischen Formen vor, die den speziellen Gegebenheiten des Applikationsfeldes entsprechen, wobei die Form des topischen Trägersystems plan oder dreidimensional, rund, oval, eckig, mit konkaven oder konvexen Aussenformen ist, oder das Trägersystem auch mit oder ohne zusätzliche Hilfsmittel vom Anwender in entsprechende Formen segmentiert werden kann.

Um insbesondere auch die Therapie von postoperativen Schmerzen im Körperinneren zu verbessern ist, in einer weiteren Ausbildung der Erfindung, das topisch wirksame Trägersystem für Lokalanästhetika aus biologisch verwertbaren oder biologisch abbaubaren Stoffen ausgebildet, wobei biologische Materialien Proteine oder Proteide, Lipide, Lipoide, Kohlenhydrate Polysaccharide oder Mischungen aus solchen Materialien sind.

Eine topisch wirksame Therapie postoperativer oder posttraumatischer Schmerzen mittels eines im, auf oder am lokalen Wundbereich applizierten Trägersystems mit Lokalanästhetika wurde bisher noch nicht beschrieben. Als ein mit dieser Erfindung erzielter Vorteil ergibt sich daher, daß mit diesem Therapieprinzip erstmals auch eine neue nichtinvasive und lokale Behandlungsmöglichkeit von postoperativen oder posttraumatischer Schmerzen ergibt.

Die topisch wirksame Therapie mit Lokalanästhetika in einem Trägersystem ermöglicht eine lokal gezielte und dauerhafte therapeutische Beeinflussung terminal und funktionell vernetzter Nervenbahnen, somit einem lokalen neuronalen Netzwerk, im oberflächlich nozizeptiven Bereich. Daher besteht keine Erfordnis zu einer Wirsktoff-Distribution im gesamten Körper, außerhalb der traumatisierten Region.

Daher kann auch mit nur geringen topisch wirksamen Dosierungen vorgegangen werden. Eine systemische Gefährdung, wie sie bei den üblichen oralen oder injektablen Analgetika, Antiphlogistika, Muskelrelaxantien oder Opioiden gegeben ist, wird vermieden, da die Lokalanästhetika, z.B. Lidocain, beim Freisetzungsvorgang u.a. auch weit überwiegend metabolisiert werden, so daß auch keine systemischen Wirkspiegel und Organbelastungen nichtbetroffener Organe auftreten.

Die Therapie ist über geringere Wirkstoff-Dosen steuerbar und länger anhaltend haltend und kann zudem nach Bedarf durch Entfernung des Trägersystems auch jederzeit unterbrochen werden.

Insgesamt weist die topisch wirksame Therapie der Schmerzsymptomatik bei postoperativen oder posttraumatischen Schmerzen daher nicht die unerwünschten Nebeneffekte auf wie die systemischer Therapien mit u.a. Analgetika, Antiphlogistika, Muskelrelaxantien, Opioiden, da die Erfordernis vorheriger Wirksstoff-Distribution über den gesamten Körper vermieden wird, mit der sonst alle anderen Organe und Organsysteme belastet werden. Zudem können hierdurch auch stärkere Wirkstoffe, in verschiedenen Fällen z.B. auch Opioide eingespart werden.

Lokalanästhetika verfügen zudem auch über eine gute lokale Gewebsverträglichkeit, partiell über eine Bakterizidie. Sie haben, über einen Einfluss auf die lokale Gefässinnervation, auch noch einen eher therapeutisch positiven Einfluss auf Wundheilungs- und Entzündungsvorgänge. Mit der nichtinvasiven, wie auch noch länger wirksamen Form, können dem Patienten zudem Schmerzen durch andere sonst erforderliche systemische oder invasive Zusatzanwendungen erspart werden. Durch die gezielte Therapie der Wundareale können andere Schmerzmittel eingespart werden, so daß sich, neben den medizinischen Vorteilen, zusätzlich auch kostensparende Effekte für die Therapie ergeben.

Als Beispiel für eine technisch geeignete Ausgestaltung für diese Therapie mit einem topisch wirksamen Trägersystem für Lokalanästhetika bei Therapie von Schmerzen äußerer Verletzungen, z.B, bei stumpfen Traumen der Extremitäten, wird auf Beschreibungen technischer Trägersysteme US-No.4,765,986, EP 0205974, DE 3716575.5, DE 3811564.6 verwiesen, ohne es auf diese beschriebenen Techniken beschränken zu wollen.

Für Anwendung dieser neuen topisch wirksamen Schmerztherapie z.B. im operativen Bereich mit biologisch abbaubaren oder biologisch verwertbaren Materialien sind als beispielhafte Trägerysteme z.B. Lipid-Formulierungen sinnvoll wie sie in US.-Pat. 5120710, EP 0404748 oder JP 2-156611 beschrieben sind, ohne es auf diese beschriebenen Techniken beschränken zu wollen.
Als Therapie postoperativer oder posttraumatischer Wundschmerzen werden geeignete topisch wirksame Trägersysteme eingesetzt, die mit therapeutisch geeigneten Dosen von Lokalanästhetika beladen sind und die enthaltenen Lokalanästhetika topisch gezielt in, auf oder an das Wundgebiet bringen, aus biologischen oder künstlichen Materialien oder einer Kombination von beiden bestehen sowie über zusätzliche Mechanismen zur Kontrolle der Freisetzung der enthaltenen Lokalanästhetika verfügen können und wobei sich das zu therapierende Wundgebiet entweder im Inneren des Körpers oder an der Körperoberfläche befindet.

Die Therapie mit geeigneten topisch wirksamen Trägersystemen mit Lokalanästhetika stellt eine neue nichtinasive, nebenwirkungsarme und effektive Behandlungsmöglichkeit postoperativer oder posttraumatischer Wundschmerzen dar, die neben den medizinischen Vorteilen auch kostensparende Therapievorteile ermöglicht.

## Patentansprüche

1. Methode und Zusammensetzung einer topisch wirksamen Therapie postoperativer oder posttraumatische Wundschmerzen, dadurch gekennzeichnet, daß als Therapie geeignete topisch wirksame Trägersysteme mit Lokalanästhetika eingesetzt werden, die mit therapeutisch geeigneten Dosen von Lokalanästhetika beladen sind, die enthaltenen Lokalanästhetika topisch gezielt in, auf oder an das Wundgebiet bringen, aus biologischen oder künstlichen Materialien oder einer Kombination von beidem bestehen sowie über zusätzliche Mechanismen zur Kontrolle der Freisetzung der enthaltenen Lokalanästhetika verfügen können, und sich das zu therapierende Wundgebiet entweder im Inneren des Körpers oder an der Körperoberfläche befindet.

2. Methode und Zusammensetzung nach vorhergehendem Anspruch, dadurch gekennzeichnet, daß Lokalanästhetika vom Amid- oder Ester-Typ eingesetzt sind, insbesondere Lidocain, Tetracain, Bupivacain, Prilocain, Mepivacain, Etidocain sowie Procain und Benzocain, wobei diese Stoffe in Konzentrationsbereichen von 0,5%-40% vorliegen.

3. Methode und Zusammensetzung nach vorhergehenden Ansprüchen, dadurch gekennzeichnet, daß in dem eingesetzten topisch wirksamen Trägersytem 2 Lokalanästhetika mit unterschiedlicher Pharmakodynamik und Pharmakokinetik kombiniert sind, wobei diese Einzelstoffe in solchen Konzentrationen vorliegen, daß die Gesamkonzentration beider Wirkstoffe nicht mehr als 40% beträgt.

4. Methode und Zusammensetzung nach vorhergehenden Ansprüchen, dadurch gekennzeichnet, daß das das topisch wirksame Trägersystem in solchen geometrischen Formen vorliegt, die den speziellen Gegebenheiten des Applikationsfeldes entsprechen, wobei die Form des topischen Trägersystems plan oder dreidimensional, rund, oval, eckig, mit konkaven oder konvexen Aussenformen ist, oder das Trägersystem auch mit oder ohne zusätzliche Hilfsmittel vom Anwender in entsprechende Formen segmentiert werden kann.

5. Methode und Zusammensetzung nach vorhergehenden Ansprüchen, dadurch gekennzeichnet, daß das topisch wirksame Trägersystem für Lokalanästhetika aus biologisch verwertbaren oder biologisch abbaubaren Stoffen ausgebildet ist, wobei biologische Materialien Proteine, Proteide, Lipide, Lipoide, Kohlenhydrate, Polysaccharide oder Mischungen aus solchen Materialien sind.
